(19) **Europäisches Patentamt / European Patent Office / Office européen des brevets**

(11) **EP 4 513 170 A1**

(12) **EUROPEAN PATENT APPLICATION**
published in accordance with Art. 153(4) EPC

(43) Date of publication:
**26.02.2025 Bulletin 2025/09**

(21) Application number: **23791738.0**

(22) Date of filing: **10.04.2023**

(51) International Patent Classification (IPC):
*G01N 21/3577* (2014.01)    *G01N 21/65* (2006.01)

(52) Cooperative Patent Classification (CPC):
**G16B 40/10; G01N 21/3577; G01N 21/65; G16B 50/30**

(86) International application number:
**PCT/JP2023/014606**

(87) International publication number:
**WO 2023/204089 (26.10.2023 Gazette 2023/43)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **18.04.2022 JP 2022068370**

(71) Applicant: **Nitto Denko Corporation Ibaraki-shi, Osaka 567-8680 (JP)**

(72) Inventors:
• **YOSHIDA, Kei**
  **Ibaraki-shi, Osaka 567-8680 (JP)**

• **KIGAWA, Yoichi**
  **Ibaraki-shi, Osaka 567-8680 (JP)**
• **SUGIURA, Shinsuke**
  **Ibaraki-shi, Osaka 567-8680 (JP)**
• **MAETA, Eri**
  **Ibaraki-shi, Osaka 567-8680 (JP)**
• **MATSUNAMI, Jun**
  **Ibaraki-shi, Osaka 567-8680 (JP)**

(74) Representative: **Hoffmann Eitle Patent- und Rechtsanwälte PartmbB Arabellastraße 30 81925 München (DE)**

Remarks:
The complete document including Reference Table(s) and the Sequence Listing(s) can be downloaded from the EPO website

(54) **NUCLEOSIDE PHOSPHOROAMIDITE IDENTIFICATION SYSTEM, AND NUCLEOSIDE PHOSPHOROAMIDITE IDENTIFICATION METHOD AND PROGRAM**

(57) A nucleoside phosphoramidite identifying system for improving a nucleoside phosphoramidite identification accuracy includes a memory unit configured to store spectra of solutions of a plurality of different nucleoside phosphoramidites, a detecting unit configured to detect a spectrum of a solution of a nucleoside phosphoramidite, and an identifying unit configured to identify the nucleoside phosphoramidite based on cosine similarity between the spectra stored in the memory unit and the spectrum detected by the detecting unit.

## Description

Technical Field

The present invention relates to a nucleoside phosphoramidite identifying system, a nucleoside phosphoramidite identifying method, and a program.

Background Art

There is a nucleic acid synthesizing device configured to synthesize an oligonucleotide by binding nucleoside phosphoramidites according to sequence information. For example, Patent Document 1 discloses a nucleic acid synthesizing device including a deprotection unit, a coupling unit, an oxidizing/thiolation unit, a capping unit, and a washing unit, wherein a plurality of reaction containers are conveyed to these units according to a synthesis scheme for a desired nucleic acid sequence.

There is a real-time monitoring technique for detecting errors in a nucleic acid synthesizing process. For example, Non-Patent Document 1 discloses a technique for real-time monitoring of an acetonitrile solution of a nucleoside phosphoramidite delivered to a reaction container, using an infrared spectroscope. The technique described in Non-Patent Document 1 identifies the type of the delivered nucleoside phosphoramidite by analyzing an infrared absorption spectrum, which is detected by the infrared spectroscope, by a Partial Least Squares Discriminant Analysis (PLS-DA) (hereinafter, may also be referred to as "PLS discrimination method").

Citation List

Patent Document

[Patent document 1] International Publication No. WO 2020/210476

Non-Patent Document

[Non-Patent Document 1] John-David McElderry, Daniel Hill, Elliott Schmitt, Xiaoye Su, and Jessica Stolee, "In-line Phosphoramidite Identification by FTIR to Support Real-Time Oligonucleotide Sequence Confirmation," Organic Process Research & Development 2021, 25, 2, 262-270.

Summary of Invention

Problem to be Solved by Invention

However, the PLS discrimination method has a problem that the nucleoside phosphoramidite discrimination accuracy is low. Particularly, there is a tendency that the lower the concentration of a nucleoside phosphoramidite, the more likely a discrimination is affected by noise, and the more often an erroneous discrimination result occurs.

In view of the technical problem described above, an object of an embodiment of the present invention is to improve the nucleoside phosphoramidite identification accuracy.

Means for Solving Problem

To solve the problem described above, a nucleoside phosphoramidite identifying system according to an embodiment of the present invention includes a memory unit configured to store spectra of a plurality of different nucleoside phosphoramidite solutions, a detecting unit configured to detect a spectrum of a nucleoside phosphoramidite solution, and an identifying unit configured to identify a nucleoside phosphoramidite based on cosine similarity between the spectra stored in the memory unit and the spectrum detected by the detecting unit.

Advantageous Effects of Invention

According to an embodiment of the present invention, it is possible to improve the nucleoside phosphoramidite identification accuracy.

Brief Description of Drawings

[FIG. 1] FIG. 1 is a diagram illustrating an example of an overall configuration of a nucleoside phosphoramidite identifying system.

[FIG. 2] FIG. 2 is a diagram illustrating an example of a hardware configuration of a nucleic acid synthesizing device.

[FIG. 3] FIG. 3 is a diagram illustrating an example of a hardware configuration of a computer.

[FIG. 4] FIG. 4 is a diagram illustrating an example of a functional configuration of a nucleoside phosphoramidite identifying system.

[FIG. 5] FIG. 5 is a drawing illustrating an example of a process flow of a nucleoside phosphoramidite identifying method.

[FIG. 6] FIG. 6 is a drawing illustrating an example of an infrared absorption spectrum.

[FIG. 7] FIG. 7 is a drawing illustrating an example of a result display screen.

[FIG. 8] FIG. 8 is a drawing illustrating an example of changes in the concentration of a nucleoside phosphoramidite solution.

[FIG. 9] FIG. 9 is a drawing illustrating examples of Raman spectra.

[FIG. 10] FIG. 10 is a diagram illustrating an example of a hardware configuration of a nucleic acid synthesizing device according to a third embodiment.

[FIG. 11] FIG. 11 is a drawing illustrating an example of a process flow of a nucleoside phosphoramidite identifying method according to the third embodiment.

[FIG. 12] FIG. 12 is a drawing indicating experimental data.

[FIG. 13] FIG. 13 is a drawing indicating identification results in Example 1.

[FIG. 14] FIG. 14 is a drawing indicating identification results in Comparative Example 1.

[FIG. 15] FIG. 15 is a drawing indicating identification results in Example 2.

[FIG. 16] FIG. 16 is a drawing indicating identification results in Example 3.

[FIG. 17] FIG. 17 is a drawing indicating identification results in Example 4.

Description of Embodiments

Each embodiment of the present invention will be described below with reference to the attached drawings. In the present specification and drawings, overlapping descriptions about components that have substantially the same function and configuration will be omitted by denoting them by the same reference numeral.

[First embodiment]

A first embodiment of the present invention is a nucleoside phosphoramidite identifying system configured to identify the type of a nucleoside phosphoramidite delivered to a column in a nucleic acid synthesizing device. The nucleoside phosphoramidite identifying system according to the present embodiment is configured to detect an infrared absorption spectrum from an acetonitrile solution containing a nucleoside phosphoramidite (hereinafter, may also be represented as "nucleoside phosphoramidite solution"), using an infrared spectroscope. The nucleoside phosphoramidite identifying system is also configured to identify the type of the delivered nucleoside phosphoramidite based on cosine similarity between the detected infrared absorption spectrum and previously stored infrared absorption spectra.

The method for detecting a spectrum from a nucleoside phosphoramidite solution is not limited to an infrared spectroscope. For example, a Raman spectroscope may be used instead of an infrared spectroscope. A configuration using a Raman spectroscope will be described in a second embodiment. In the present embodiment, any other spectroscope may also be used. However, it is preferable to use an infrared spectroscope or a Raman spectroscope for nucleoside phosphoramidite identification, although any other spectroscope may be used.

Cosine similarity is an indicator of similarity between two vectors. Specifically, cosine similarity is a cosine value of an angle formed between two vectors. For example, the cosine similarity between a vector "$\vec{a}=(a_1, a_2, ..., a_n)$" and a vector "$\vec{b}=(b_1, b_2, ..., b_n)$" is represented by Formula (1).

In each embodiment of the present invention, a vector "$\vec{a}$" and a vector "$\vec{b}$" are vectors representing the total wavenumber in the analyzing wavenumber range (hereinafter, may also be represented as the measurement wavenumber range) of an infrared absorption spectrum or a Raman spectrum. The vector "$\vec{a}$" is data regarding a spectrum (reference) of a nucleoside phosphoramidite, the type of which has been previously determined. The vector "$\vec{b}$" is data regarding a detected spectrum of a nucleoside phosphoramidite, the type of which is unknown. Here, a maximum cosine similarity is obtained between a reference and a sample between which $\cos(\vec{a},\vec{b})$ represented by Formula (1) is the closest to 1.

For example, cos(a→,b→) is calculated between a detected spectrum of an unknown nucleoside phosphoramidite and the spectrum of each of various types of nucleoside phosphoramidites (containing adenine, thymine, guanine, and cytosine as bases) previously obtained as references. When cos(a→,b→) calculated with respect to the spectrum of an adenine nucleoside phosphoramidite is the closest to 1 (i.e., the cosine similarity is the maximum), the detected spectrum is determined to be of an adenine nucleoside phosphoramidite.

[Math. 1]

$$\cos\left(\vec{a}, \vec{b}\right) = \frac{\sum_{i=1}^{n} a_i b_i}{\sqrt{\sum_{i=1}^{n} a_i^2} \cdot \sqrt{\sum_{i=1}^{n} b_i^2}} \quad \cdots (1)$$

The nucleoside phosphoramidite identifying system according to the present embodiment is also configured to check a nucleoside phosphoramidite identification result against the sequence information of a synthesizing-target oligonucleotide. The nucleoside phosphoramidite identifying system according to the present embodiment is configured to display the identification result and the checking result in association with each other in real time on a display device such as a display.

Moreover, the nucleoside phosphoramidite identifying system according to the present embodiment is configured to give an alert or control the nucleic acid synthesizing device to stop delivery of a nucleoside phosphoramidite, when an error is detected from the checking result.

<Overall configuration of nucleoside phosphoramidite identifying system>

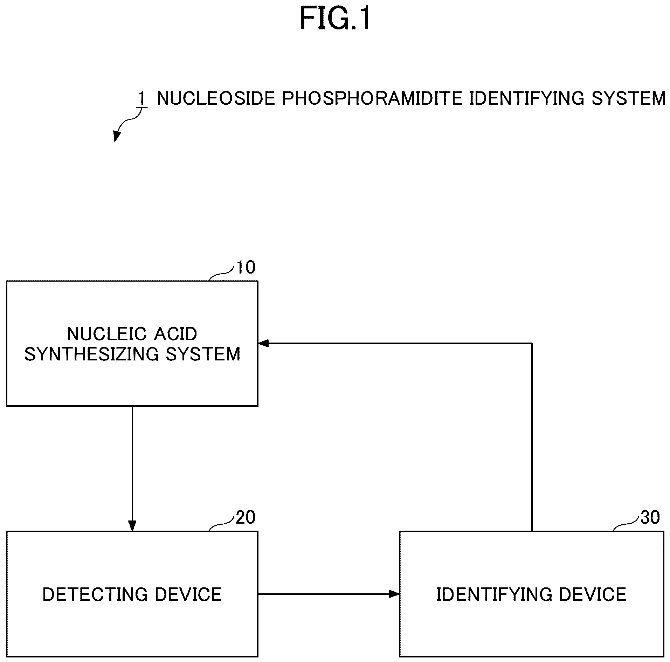

First, the overall configuration of the nucleoside phosphoramidite identifying system according to the present embodiment will be described with reference to FIG. 1. FIG. 1 is a block diagram illustrating an example of the overall configuration of the nucleoside phosphoramidite identifying system according to the present embodiment.

As illustrated in FIG. 1, the nucleoside phosphoramidite identifying system 1 according to the present embodiment includes a nucleic acid synthesizing device 10, a detecting device 20, and an identifying device 30. The detecting device 20 is optically connected to a flow cell provided on a tube of the nucleic acid synthesizing device 10. The identifying device 30 is electrically connected to the nucleic acid synthesizing device 10 and the detecting device 20.

The nucleic acid synthesizing device 10 is a nucleic acid synthesizing device configured to synthesize an oligonucleotide. The nucleic acid synthesizing device 10 includes a plurality of tanks in which acetonitrile solutions containing various types of nucleoside phosphoramidites used for synthesizing oligonucleotides, a solution containing an activating agent, a solution containing a deprotection agent, a solution containing an oxidizing agent or a thiolation agent, a solution containing a capping agent, an acetonitrile for washing, and an amine wash reaction solution are stored, and a column in which an oligonucleotide is synthesized. The tanks and the column are connected through a tube via a pump. The pump is configured to perform a control to deliver a nucleoside phosphoramidite solution to the column from any one of the tanks according to sequence information of an oligonucleotide. The tube that connects the pump and the column is provided with a flow cell to which various measuring instruments are connected.

The detecting device 20 is a measuring instrument configured to detect a spectrum of a nucleoside phosphoramidite solution in a flow cell provided in the nucleic acid synthesizing device 10. The detecting device 20 according to the present embodiment is an infrared spectroscope configured to detect an infrared absorption spectrum of a nucleoside phosphoramidite solution before being delivered to the column, by irradiating the nucleoside phosphoramidite solution with infrared rays.

The identifying device 30 is an information processing device such as a Personal Computer (PC), a workstation, and a server configured to identify the type of a nucleoside phosphoramidite to be delivered to the column based on the infrared absorption spectrum of the nucleoside phosphoramidite solution detected by the detecting device 20. A memory unit of the identifying device 30 previously stores, as references, infrared absorption spectra detected by the detecting device 20 from various types of nucleoside phosphoramidite solutions. The identifying device 30 identifies the type of the nucleoside phosphoramidite to be delivered to the column based on the cosine similarity between the infrared absorption spectra, which are the references stored in the memory unit, and the infrared absorption spectrum of the nucleoside phosphoramidite solution, which is the detection target detected by the detecting device 20.

The overall configuration of the nucleoside phosphoramidite identifying system 1 illustrated in FIG. 1 is an example, and there are possibly various system configuration examples depending on the use or purpose. For example, the identifying device 30 may be realized by a plurality of computers, and may be realized as a cloud computing service. Moreover, for example, the nucleoside phosphoramidite identifying system 1 may be realized by a standalone information processing device comprehensively equipped with the functions, which the detecting device 20 and the identifying device 30 must have.

<Hardware configuration of nucleoside phosphoramidite identifying system>

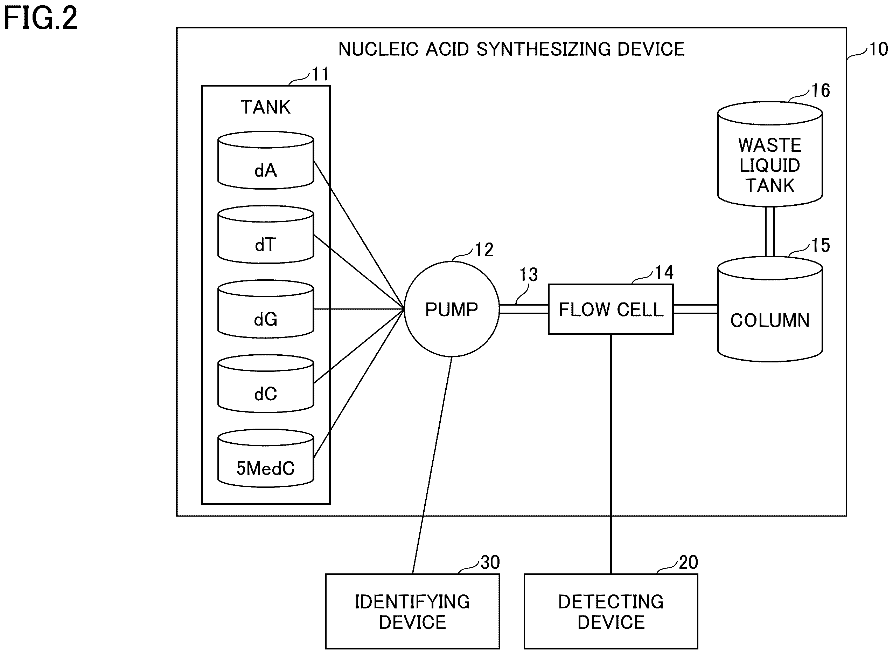

Next, the hardware configuration of the nucleoside phosphoramidite identifying system according to the present embodiment will be described with reference to FIG. 2.

<<Hardware configuration of nucleic acid synthesizing device>>

FIG. 2 is a block diagram illustrating an example of the hardware configuration of the nucleic acid synthesizing device 10 according to the present embodiment. As illustrated in FIG. 2, the nucleic acid synthesizing device 10 according to the present embodiment includes a tank 11, a pump 12, a tube 13, a flow cell 14, a column 15, and a waste liquid tank 16.

The tank 11 is configured to store acetonitrile solutions containing nucleoside phosphoramidites. The tank 11 is formed of a plurality of tanks, and the tanks each store the acetonitrile solution containing one type of a nucleoside phosphoramidite, the solution containing the activating agent (unillustrated), the solution containing the deprotection agent (unillustrated), the solution containing the oxidizing agent (unillustrated) or the thiolation agent (unillustrated), the solution containing the capping agent (unillustrated), the acetonitrile for washing (unillustrated), and the amine wash reaction solution (unillustrated), respectively. In the present embodiment, five types of nucleoside phosphoramidites containing, as DNA bases, adenine (hereinafter, represented as "dA"), thymine (hereinafter, represented as "dT"), guanine (hereinafter, represented as "dG"), cytosine (hereinafter, represented as "dC"), and 5-methyl-cytosine (hereinafter, represented as "5MedC") are stored in the tank 11. Nucleoside phosphoramidites are not limited to those mentioned above, and may be other DNA modifiers, RNA, and RNA modifiers.

The pump 12 is connected to the tank 11 and the column 15 through the tube 13. The pump 12 is configured to deliver a nucleoside phosphoramidite solution from the tank 11 to the column 15. The pump 12 is controlled to deliver a nucleoside phosphoramidite solution of any type to the column 15 in order according to the sequence information of an oligonucleotide.

The flow cell 14 is provided on the tube 13 that connects the pump 12 and the column 15. The flow cell 14 is configured such that various types of measuring instruments can be optically connected to the flow cell 14.

The column 15 is a reaction container in which the nucleoside phosphoramidites, the activating agent, the deprotection agent, the oxidizing agent or the thiolation agent, and the capping agent that are delivered from the tank 11 via the pump 12 react with each other, to produce an oligonucleotide. In the column 15, for example, a nucleic acid synthesizing process including such steps as deprotection, coupling, oxidization or thiolation, and capping is performed.

The waste liquid tank 16 is connected to the column 15 through the tube 13. The solution exhausted in the column 15 is discarded into the waste liquid tank 16.

The hardware configuration of the nucleic acid synthesizing device 10 illustrated in FIG. 2 is an example. The nucleic acid synthesizing device 10 according to the present embodiment may have any hardware configuration, so long as a spectrum can be detected from a nucleoside phosphoramidite solution to be delivered to the column 15.

<<Hardware configuration of computer>>

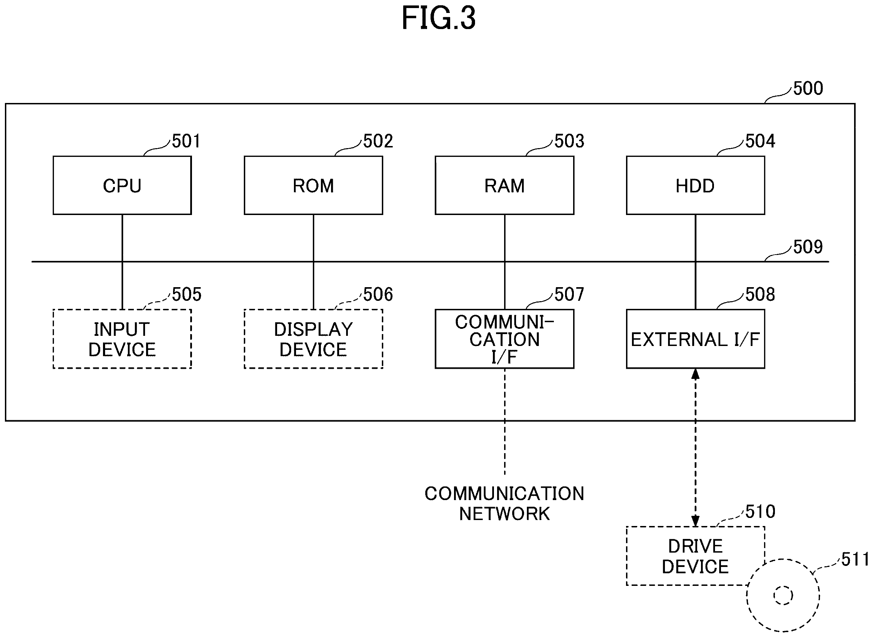

The detecting device 20 and the identifying device 30 according to the present embodiment are realized by, for example, a computer. FIG. 3 is a block diagram illustrating an example of the hardware configuration of a computer 500 according to the present embodiment.

As illustrated in FIG. 3, the computer 500 includes a Central Processing Unit (CPU) 501, a Read Only Memory (ROM) 502, a Random Access Memory (RAM) 503, a Hard Disk Drive (HDD) 504, an input device 505, a display device 506, a communication interface (I/F) 507, and an external I/F 508. The CPU 501, the ROM 502, and the RAM 503 form what is generally referred to as a computer. The respective hardware pieces of the computer 500 are mutually connected through a bus line 509. The input device 505 and the display device 506 may be used while being connected to the external I/F 508.

The CPU 501 is an operating device configured to realize controls and functions of the entirety of the computer 500 by reading out programs and data onto the RAM 503 from a memory device such as the ROM 502 or the HDD 504 and executing processes.

The ROM 502 is an example of a nonvolatile semiconductor memory (memory device) that can retain programs and data even when power supply is cut. The ROM 502 functions as a main memory device configured to store, for example, various programs and data necessary for the CPU 501 to execute various programs installed on the HDD 504.

Specifically, the ROM 502 stores boot programs such as Basic Input/Output System (BIOS) and Extensible Firmware Interface (EFI) that are executed when the computer 500 is started, and data such as Operating System (OS) settings and network settings.

The RAM 503 is an example of a volatile semiconductor memory (memory device) from which programs and data are erased when power supply is cut. The RAM 503 is, for example, a Dynamic Random Access Memory (DRAM) or a Static Random Access Memory (SRAM). The RAM 503 provides a work area in which various programs installed on the HDD 504 are deployed when executed by the CPU 501.

The HDD 504 is an example of a nonvolatile memory device storing programs and data. The programs and data stored in the HDD 504 include OS, which is basic software for controlling the entirety of the computer 500, and applications that provide various functions on the OS. The computer 500 may employ a memory device using a flash memory as a memory medium (e.g., a Solid State Drive (SSD)), instead of the HDD 504.

The input device 505 is, for example, a touch panel, operation keys or buttons, or a keyboard or a mouse by which a user enters various signals, or a microphone by which a user enters sound data such as sound or voice.

The display device 506 is formed of a liquid crystal or organic Electro-Luminescence (EL) display configured to display a screen, and a loudspeaker configured to output sound data such as sound and voice.

The communication I/F 507 is an interface configured to connect to a communication network in order that the computer 500 can perform data communication.

The external I/F 508 is an interface to an external device. An example of the external device is a drive device 510.

The drive device 510 is a device configured for a recording medium 511 to be set therein. The recording medium 511 meant here encompasses media configured to record information optically, electrically, or magnetically, such as a CD-ROM, a flexible disk, and a magneto-optical disk. The recording medium 511 may also encompass, for example, semiconductor memories configured to record information electrically, such as a ROM and a flash memory. Hence, the computer 500 can perform either or both of reading from and writing into the recording medium 511 via the external I/F 508.

Various programs to be installed on the HDD 504 are installed by, for example, a distributed recording medium 511 being set in the drive device 510 connected to the external I/F 508, and various programs recorded in the recording medium 511 being read out by the drive device 510. Alternatively, various programs to be installed on the HDD 504 may be installed by being downloaded from any other network different from the communication network via the communication I/F 507.

<Functional configuration of nucleoside phosphoramidite identifying system>

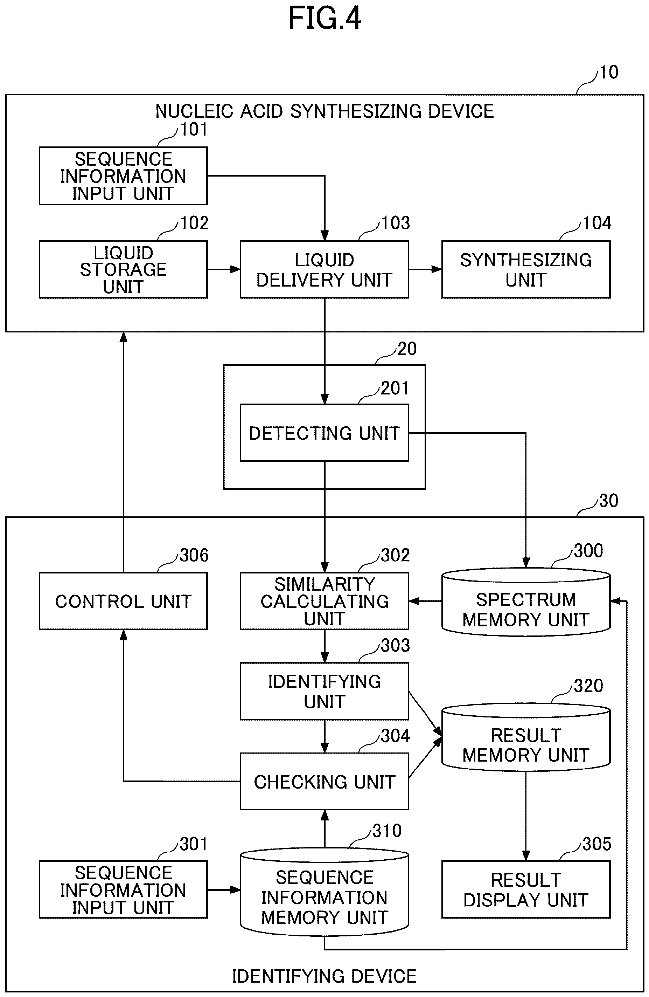

Next, the functional configuration of the nucleoside phosphoramidite identifying system according to the present embodiment will be described with reference to FIG. 4. FIG. 4 is a block diagram illustrating an example of the functional configuration of the nucleoside phosphoramidite identifying system according to the present embodiment.

<<Functional configuration of nucleic acid synthesizing device>>

As illustrated in FIG. 4, the nucleic acid synthesizing device 10 according to the present embodiment includes a sequence information input unit 101, a liquid storage unit 102, a liquid delivery unit 103, and a synthesizing unit 104.

The sequence information input unit 101 is configured to receive an input of nucleic acid synthesizing conditions under which the synthesizing unit 104 synthesizes an oligonucleotide (e.g., liquid delivery conditions for the nucleoside phosphoramidite solutions, the solution containing the activating agent, the solution containing the deprotection agent, the solution containing the oxidizing agent or the thiolation agent, the solution containing the capping agent, the acetonitrile for washing, and the amine wash reaction solution), and sequence information representing the nucleic acid sequence of the oligonucleotide to be synthesized by the nucleic acid synthesizing device 10 in response to an operation of a user. The sequence information is information representing the types of the bases of nucleoside phosphoramidites and the order to bind the nucleoside phosphoramidites in order to synthesize an oligonucleotide. In this case, the nucleic acid synthesizing device 10 synthesizes an oligonucleotide in accordance with the nucleic acid synthesizing conditions and the sequence information (hereinafter, may also be represented as sequence information, etc.) (hereinafter, this mode may also be represented as a nucleic acid synthesizing mode).

The sequence information input unit 101 is also configured to receive an input of a liquid delivery condition under which the liquid delivery unit 103 delivers a nucleoside phosphoramidite solution of each type individually, and individual information exclusively regarding the nucleoside phosphoramidite of the type to be delivered, in response to an operation of a user. In this case, the nucleic acid synthesizing device 10 delivers the nucleoside phosphoramidite solution of each type individually in accordance with the liquid delivery condition and the individual information (hereinafter, may also be represented as individual information, etc.) without performing nucleic acid synthesis (hereinafter, this mode may also be represented as an individual liquid delivery mode).

The liquid storage unit 102 stores the various types of nucleoside phosphoramidite solutions, the solution containing the activating agent, the solution containing the deprotection agent, the solution containing the oxidizing agent or the thiolation agent, the solution containing the capping agent, the acetonitrile for washing, and the amine wash reaction solution. The liquid storage unit 102 is realized by, for example, the tank 11 illustrated in FIG. 2.

In the nucleic acid synthesizing mode, the liquid delivery unit 103 is configured to deliver the nucleoside phosphoramidite solutions, the solution containing the activating agent, the solution containing the deprotection agent, the solution containing the oxidizing agent or the thiolation agent, the solution containing the capping agent, the acetonitrile for washing, or the amine wash reaction solution stored in the liquid storage unit 102 to the synthesizing unit 104 in order in accordance with the sequence information, etc. received by the sequence information input unit 101.

In the individual liquid delivery mode, the liquid delivery unit 103 is configured to deliver a nucleoside phosphoramidite solution stored in the liquid storage unit 102 to the synthesizing unit 104 in accordance with the individual information, etc. received by the sequence information input unit 101.

The liquid delivery unit 103 is realized by, for example, the pump 12 and the tube 13 illustrated in FIG. 2.

In the nucleic acid synthesizing mode, the synthesizing unit 104 is configured to synthesize an oligonucleotide by a predetermined nucleic acid synthesizing method using the nucleoside phosphoramidite solutions, the solution containing the activating agent, the solution containing the deprotection agent, the solution containing the oxidizing agent or the thiolation agent, the solution containing the capping agent, the acetonitrile for washing, and the amine wash reaction solution delivered by the liquid delivery unit 103. The synthesizing unit 104 is realized by, for example, the column 15 illustrated in FIG. 2.

<<Functional configuration of detecting device>>

As illustrated in FIG. 4, the detecting device 20 according to the present embodiment includes a detecting unit 201.

The detecting unit 201 is realized by a process, which the CPU 501 is caused to execute by a program deployed in the RAM 503 from the HDD 504 illustrated in FIG. 3.

The detecting unit 201 is configured to detect an infrared absorption spectrum from a nucleoside phosphoramidite solution delivered by the liquid delivery unit 103. Hereinafter, the infrared absorption spectrum detected by the detecting unit 201 may also be referred to as a "detected spectrum". The detecting unit 201 is configured to output a detected spectrum to the identifying device 30.

<<Functional configuration of identifying device>>

As illustrated in FIG. 4, the identifying device 30 according to the present embodiment includes a spectrum memory unit 300, a sequence information input unit 301, a similarity calculating unit 302, an identifying unit 303, a checking unit 304, a result display unit 305, a control unit 306, a sequence information memory unit 310, and a result memory unit 320.

The sequence information input unit 301, the similarity calculating unit 302, the identifying unit 303, the checking unit 304, the result display unit 305, and the control unit 306 are realized by processes, which the CPU 501 is caused to execute by a program deployed in the RAM 503 from the HDD 504 illustrated in FIG. 3.

The spectrum memory unit 300, the sequence information memory unit 310, and the result memory unit 320 are realized by, for example, using the HDD 504 illustrated in FIG. 3.

The spectrum memory unit 300 is configured to store infrared absorption spectra that have been previously detected as references from identifying-target nucleoside phosphoramidites in the individual liquid delivery mode. Hereinafter, the infrared absorption spectra stored in the spectrum memory unit 300 may also be referred to as "identifying-target spectra". The identifying-target spectra are stored in the spectrum memory unit 300 in association with individual information regarding various types of nucleoside phosphoramidites input via the sequence information input unit 301.

Identifying-target nucleoside phosphoramidites are nucleoside phosphoramidites of various types included in sequence information of an oligonucleotide to be synthesized by the nucleic acid synthesizing device 10. In other words, identifying-target nucleoside phosphoramidites are nucleoside phosphoramidites of various types that are stored in the liquid storage unit 102 of the nucleic acid synthesizing device 10.

The identifying-target spectra stored in the spectrum memory unit 300 for various types of nucleoside phosphoramidites may each include a plurality of infrared absorption spectra that are detected using nucleoside phosphoramidite solutions prepared at different concentrations. With this configuration, it is possible to identify also the concentrations of the acetonitrile solutions in addition to the types of the nucleoside phosphoramidites.

The sequence information input unit 301 is configured to receive an input of sequence information of an oligonucleotide for the nucleic acid synthesizing mode, or individual information of a nucleoside phosphoramidite of each type for the individual liquid delivery mode in response to an operation of a user.

The sequence information memory unit 310 is configured to store sequence information of an oligonucleotide or individual information of a nucleoside phosphoramidite of each type received by the sequence information input unit 301.

The similarity calculating unit 302 is configured to calculate the cosine similarity between each of the identifying-target spectra stored in the spectrum memory unit 300 and a detected spectrum detected by the detecting device 20.

The identifying unit 303 is configured to identify the type of a nucleoside phosphoramidite delivered by the liquid delivery unit 103 of the nucleic acid synthesizing device 10 based on the cosine similarity calculated by the similarity calculating unit 302. Specifically, the identifying unit 303 determines the identifying-target spectrum corresponding to the maximum cosine similarity among the cosine similarities calculated by the similarity calculating unit 302 (i.e., determines the identifying-target spectrum that is the most similar to the detected spectrum). Next, the identifying unit 303 determines the type of the nucleoside phosphoramidite corresponding to the determined identifying-target spectrum, as the type of the nucleoside phosphoramidite delivered.

The checking unit 304 is configured to check the nucleoside phosphoramidite identification result of the identifying unit 303 against the sequence information or the individual information stored in the sequence information memory unit 310. Specifically, the checking unit 304 first determines the correct nucleoside phosphoramidite that is supposed to be being delivered currently, according to the sequence information or the individual information. Next, the checking unit 304 determines whether the correct nucleoside phosphoramidite matches the nucleoside phosphoramidite identification result of the identifying unit 303.

The result memory unit 320 is configured to store the identification result of the identifying unit 303 and the checking result of the checking unit 304 in association with each other.

The result display unit 305 is configured to display the identification result and the checking result stored in the result memory unit 320 on a display device 506 in association with each other. Here, when the checking result indicates a mismatch, the result display unit 305 displays an alert to that effect.

The control unit 306 is configured to control the nucleic acid synthesizing device 10 to stop liquid delivery when the checking result of the checking unit 304 indicates a mismatch. Examples of the method for stopping liquid delivery include a method of closing the tube 13 such that the solution cannot flow, and a method of sending a control signal to stop the pump 12, although the method is different depending on the nucleic acid synthesizing device 10.

<Process flow of nucleoside phosphoramidite identifying method>

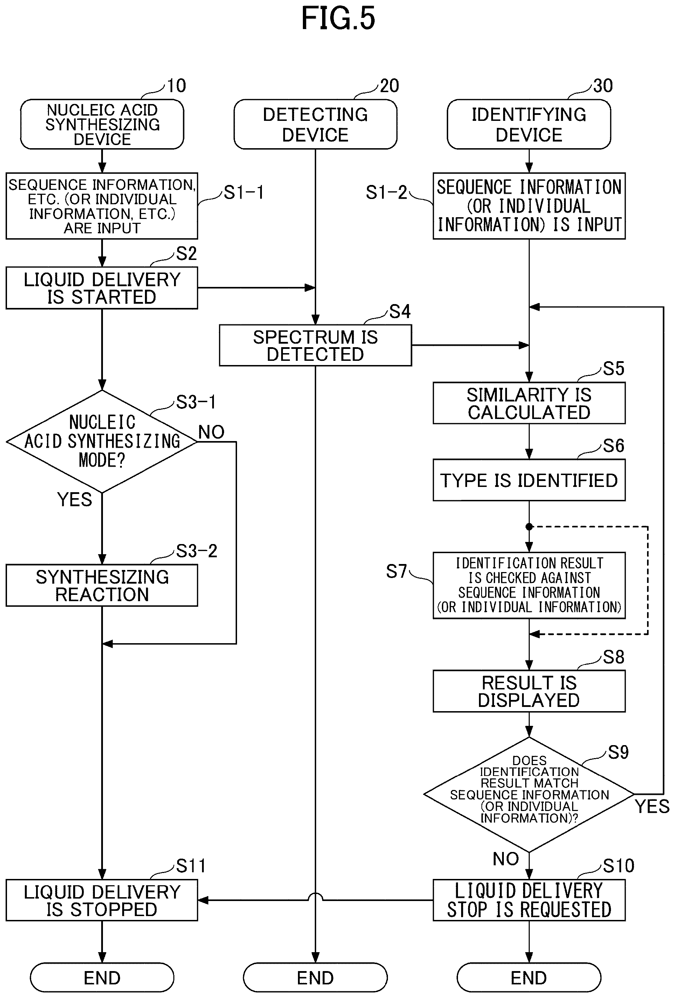

Next, the process flow of a nucleoside phosphoramidite identifying method performed by the nucleoside phosphoramidite identifying system according to the present embodiment will be described with reference to FIG. 5. FIG. 5 is a flowchart illustrating an example of the process flow of the nucleoside phosphoramidite identifying method according to the present embodiment.

In the step S1-1, the sequence information input unit 101 of the nucleic acid synthesizing device 10 receives an input of sequence information, etc. of an oligonucleotide, or individual information, etc. of a nucleoside phosphoramidite of each type in response to an operation of a user. Next, the sequence information input unit 101 sends the received sequence information, etc. or individual information, etc. to the liquid delivery unit 103.

In the step S1-2, the sequence information input unit 301 of the identifying device 30 receives an input of sequence information of an oligonucleotide or individual information of a nucleoside phosphoramidite of each type in response to an operation of the user. Next, the sequence information input unit 301 stores the received sequence information or individual information in the sequence information memory unit 310.

In the step S2, in the nucleic acid synthesizing mode, the liquid delivery unit 103 of the nucleic acid synthesizing device 10 delivers the nucleoside phosphoramidite solutions, the solution containing the activating agent, the solution containing the deprotection agent, the solution containing the oxidizing agent or the thiolation agent, the solution containing the capping agent, the acetonitrile for washing, or the amine wash reaction solution stored in the liquid storage unit 102 to the synthesizing unit 104 in order in accordance with the sequence information, etc. received from the sequence information input unit 101.

In the individual liquid delivery mode, the liquid delivery unit 103 delivers a nucleoside phosphoramidite solution stored in the liquid storage unit 102 to the synthesizing unit 104 in accordance with the individual information, etc. received from the sequence information input unit 101.

The liquid delivery unit 103 may receive sequence information of an oligonucleotide or individual information of a nucleoside phosphoramidite of each type from the sequence information input unit 301.

In the step S3-1, the synthesizing unit 104 of the nucleic acid synthesizing device 10 determines whether the mode is the nucleic acid synthesizing mode or the individual liquid delivery mode. When the mode is the nucleic acid synthesizing mode (YES), the synthesizing unit 104 forwards the flow to the step S3-**2.** When the mode is the individual liquid delivery mode (NO), the synthesizing unit 104 skips the step S3-2.

In the step S3-2, the synthesizing unit 104 of the nucleic acid synthesizing device 10 synthesizes an oligonucleotide according to a predetermined nucleic acid synthesizing method using the nucleoside phosphoramidites, the activating agent, the deprotection agent, the oxidizing agent or the thiolation agent, and the capping agent delivered by the liquid delivery unit 103.

In the step S4, the detecting unit 201 of the detecting device 20 detects an infrared absorption spectrum from a nucleoside phosphoramidite solution delivered by the liquid delivery unit 103. Next,the detecting unit 201 outputs the detected spectrum to the identifying device 30.

<<Infrared absorption spectrum>>

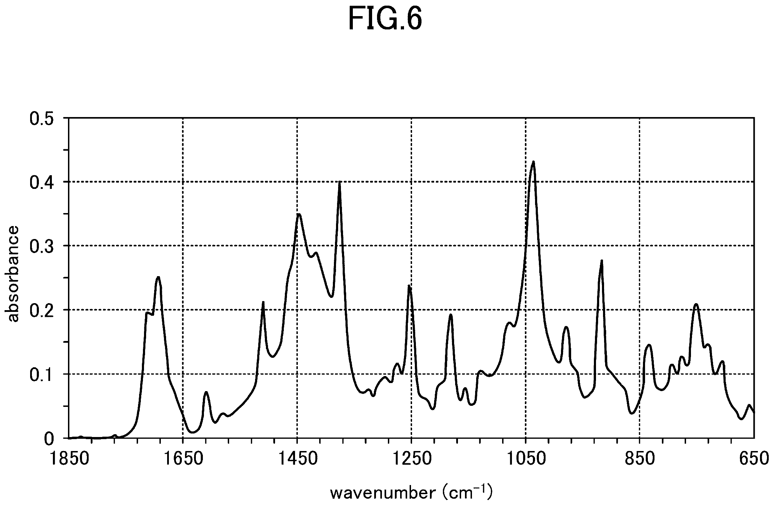

An infrared absorption spectrum will be described with reference to FIG. 6. FIG. 6 is a drawing illustrating an example of an infrared absorption spectrum.

Specifically, FIG. 6 is an example of an infrared absorption spectrum detected from a dT nucleoside phosphoramidite. The example of FIG. 6 is an infrared absorption spectrum detected from a dT nucleoside phosphoramidite-containing acetonitrile solution prepared at a concentration of 200 mM. In FIG. 6, the horizontal axis represents wavenumber, and the vertical axis represents absorbance.

The description returns to FIG. 5. In the step S5, the similarity calculating unit 302 of the identifying device 30 receives an input of the detected spectrum output by the detecting device 20. Next, the similarity calculating unit 302 reads out the identifying-target spectra stored in the spectrum memory unit 300.

Next, the similarity calculating unit 302 calculates the cosine similarity between an identifying-target spectrum and the detected spectrum, for each identifying-target spectrum. Next, the similarity calculating unit 302 sends the cosine similarities corresponding to the respective identifying-target spectra to the identifying unit 303.

In the step S6, the identifying unit 303 of the identifying device 30 receives the cosine similarities from the similarity calculating unit 302. Next, the identifying unit 303 determines the identifying-target spectrum corresponding to the maximum cosine similarity among the received cosine similarities. Next, the identifying unit 303 determines the type of the nucleoside phosphoramidite corresponding to the determined identifying-target spectrum, as the type of the nucleoside phosphoramidite delivered.

The identifying unit 303 sends the identification result indicating the type of the determined nucleoside phosphoramidite to the checking unit 304. Next, the identifying unit 303 stores the identification result in the result memory unit 320.

The identifying unit 303 need not send the identification result to the checking unit 304, and may need only to store the identification result in the result memory unit 320. In this case, the identifying unit 303 skips the step S7 and forwards the flow to the step S8.

In the step S7, the checking unit 304 of the identifying device 30 receives the identification result from the identifying unit 303. Next, the checking unit 304 determines the correct nucleoside phosphoramidite that is supposed to be being delivered currently, according to the sequence information or the individual information stored in the sequence information memory unit 310.

Next, the checking unit 304 determines whether the correct nucleoside phosphoramidite and the nucleoside phosphoramidite identification result match each other. Next, the checking unit 304 stores a checking result indicating whether the correct answer and the identification result match each other or not in the result memory unit 320.

In the step S8, the result display unit 305 of the identifying device 30 displays the identification result and the checking result that are stored in the result memory unit 320 on the display device 506 in association with each other. Here, when the checking result indicates a mismatch, the result display unit 305 displays an alert to that effect. When the checking result indicates a mismatch, the result display unit 305 may output an alerting sound from the loudspeaker of the display device 506.

When the step S7 has been skipped, the result display unit 305 displays the identification result stored in the result memory unit 320 on the display device 506.

<<Result display screen>>

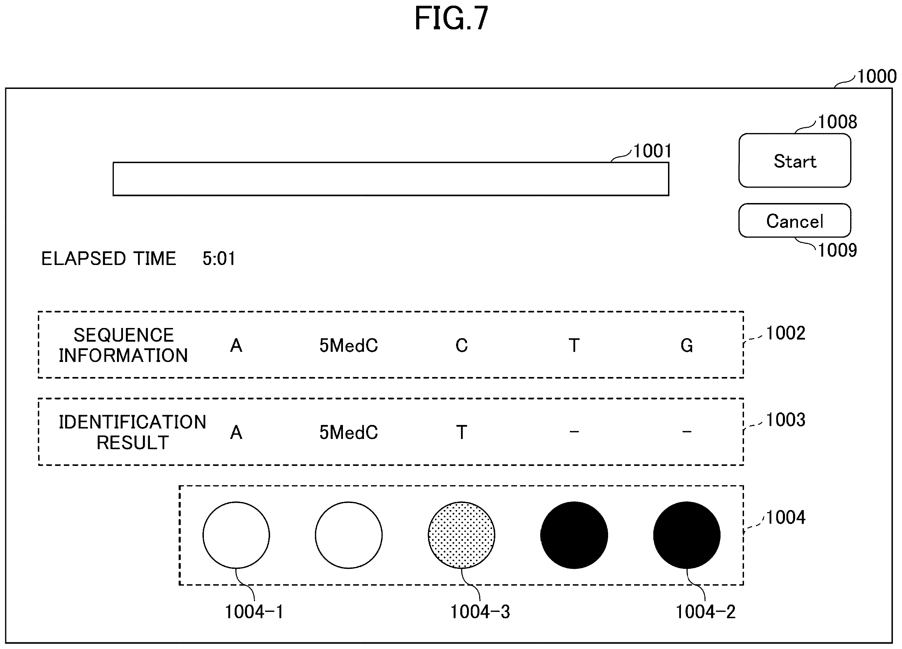

A result display screen according to the present embodiment will be described with reference to FIG. 7. FIG. 7 is a concept drawing illustrating an example of the result display screen according to the present embodiment.

As illustrated in FIG. 7, the result display screen 1000 according to the present embodiment includes a sequence information input field 1001, a sequence information display field 1002, an identification result display field 1003, a checking result display field 1004, a start button 1008, and a cancel button 1009.

The sequence information input field 1001 receives an input of sequence information of an oligonucleotide or individual information of a nucleoside phosphoramidite of each type in response to an operation of a user. When the user inputs sequence information or individual information and depresses the start button 1008, nucleoside phosphoramidite identification is started.

The sequence information display field 1002 displays a predetermined number of nucleoside phosphoramidites that are to be delivered from that instant. In the example in FIG. 7, it is indicated that dA, 5MedC, dC, dT, and dG need to be delivered in this order.

The identification result display field 1003 displays identification results corresponding to each sequence information in order. In the example in FIG. 7, it is indicated that dA, 5MedC, and dT have been identified in this order. "-" indicates that identification has not been performed yet.

The checking result display field 1004 displays checking results corresponding to the respective identification results in order. The checking result display field 1004 displays the checking results by color variation. In the example in FIG. 7, white represents a match, halftone represents a mismatch, and black represents "not identified yet". For color variation, any colors may be used so long as the user can easily discern the colors. For example, "match" may be set to green, "mismatch" may be set to red, and "not identified yet" may be set to gray. The display style of the checking results is not limited to color variation, and any display style may be used so long as the user can easily understand the checking results by the display style.

When the step S7 has been skipped, the checking result display field 1004 is not displayed on the result display screen 1000.

The description returns to FIG. 5. In the step S9, the checking unit 304 of the identifying device 30 determines whether the checking result in the step S7 indicates a match or not (i.e., whether the identification result matches the sequence information or the individual information). When the checking result indicates a match (YES), the checking unit 304 returns the flow to the step S5, in which a detected spectrum of a nucleoside phosphoramidite to be delivered next is awaited. When the checking result indicates a mismatch (NO), the checking unit 304 forwards the flow to the step S10.

In the step S10, the control unit 306 of the identifying device 30 controls the nucleic acid synthesizing device 10 to stop liquid delivery. For example, this control is a control to close the tube 13 of the nucleic acid synthesizing device 10. For example, this control may also be a control to send a signal to stop the pump 12 of the nucleic acid synthesizing device 10.

In the step S11, the liquid delivery unit 103 of the nucleic acid synthesizing device 10 stops liquid delivery in response to the control from the control unit 306. For example, when the tube 13 is closed, the nucleic acid synthesizing device 10 senses a liquid delivery abnormity and automatically stops liquid delivery. For example, when a signal to stop the pump 12 is received, the nucleic acid synthesizing device 10 stops the pump 12 in response to the stop signal.

<Effects of first embodiment>

The nucleoside phosphoramidite identifying system according to the present embodiment identifies the type of a nucleoside phosphoramidite based on the cosine similarity between an infrared absorption spectrum detected from a nucleoside phosphoramidite solution and infrared absorption spectra that have been previously detected from various types of nucleoside phosphoramidite solutions and stored as references.

Existing techniques identify the type of a nucleoside phosphoramidite based on the PLS discrimination method. However, the existing techniques have a problem that the identification accuracy is low when the concentration of the nucleoside phosphoramidite is low. One factor behind this is that detection of a spectrum from a lowconcentration nucleoside phosphoramidite tends to be affected by noise.

Particularly, when the nucleic acid synthesizing device delivers a nucleoside phosphoramidite solution from the tank to the column, the concentration of the nucleoside phosphoramidite solution changes in the tube. This is because the interior of the tube needs to be washed with acetonitrile between the steps of the nucleic acid synthesizing method, so the nucleoside phosphoramidite solution is delivered by being injected into the tube filled with acetonitrile.

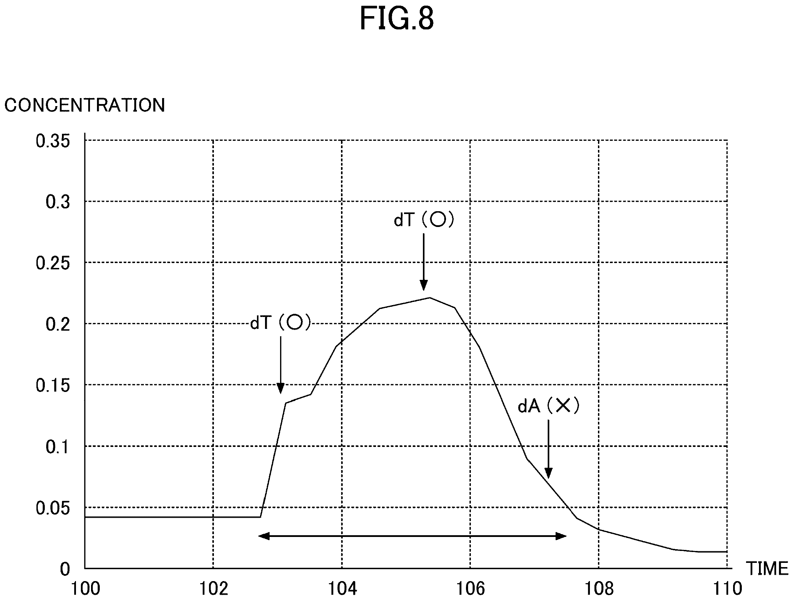

FIG. 8 is a graph plotting the results of measuring the concentration of a dT nucleoside phosphoramidite solution at a spectral intensity of 1,250 cm$^{-1}$ during liquid delivery, and the results of identifying the measured results by an existing PLS discrimination method. As plotted in FIG. 8, the concentration of the nucleoside phosphoramidite solution gradually decreases near the end of the liquid delivery. Hence, the existing PLS discrimination method erroneously determines the solution as dA. As can be seen, according to the existing PLS discrimination method, there is a tendency that erroneous determination occurs more often when determination is performed in a concentration-decreased state near the end of the liquid delivery.

The PLS discrimination method calculates the similarity by focusing on, for example, the position and height of a spectral peak. Hence, if a peak appears at a different position or height under the effects of noise, the likelihood of erroneous identification increases. On the other hand, according to the cosine similarity, the focus is on the spectral shape when the similarity is calculated. Hence, even if a peak appears at a different position or height under the effects of noise, a correct identification can be obtained so long as a significantly different spectral shape is not detected.

Accordingly, the nucleoside phosphoramidite identifying system according to the present embodiment has a nucleoside phosphoramidite identification accuracy improved over the existing techniques.

[Second embodiment]

The first embodiment is configured to identify the type of a nucleoside phosphoramidite based on an infrared absorption spectrum detected from a nucleoside phosphoramidite solution, using an infrared spectroscope as the detecting device 20. The second embodiment is configured to identify the type of a nucleoside phosphoramidite based on a Raman spectrum detected from a nucleoside phosphoramidite solution, using a Raman spectroscope as the detecting device 20.

The nucleoside phosphoramidite identifying system 1 according to the present embodiment will be described below, mainly regarding differences from the nucleoside phosphoramidite identifying system 1 according to the first embodiment.

<Functional configuration of nucleoside phosphoramidite identifying system>

The detecting device 20 according to the present embodiment is a Raman spectroscope configured to decompose Raman-scattered light, which is generated in response to irradiation of a nucleoside phosphoramidite solution delivered to a column with laser, into spectra.

The detecting unit 201 according to the present embodiment is configured to detect a Raman spectrum from a nucleoside phosphoramidite solution delivered by the liquid delivery unit 103. The detecting unit 201 is also configured to output the detected Raman spectrum to the identifying device 30.

The spectrum memory unit 300 according to the present embodiment is configured to store Raman spectra previously detected from identifying-target nucleoside phosphoramidites. Hereinafter, the Raman spectra stored in the spectrum memory unit 300 may also be referred to as "identifying-target spectra".

The similarity calculating unit 302 according to the present embodiment is configured to calculate the cosine similarity between each Raman spectrum stored in the spectrum memory unit 300 and a Raman spectrum detected by the detecting device 20.

The identifying unit 303 according to the present embodiment is configured to identify the type of the nucleoside phosphoramidite delivered by the liquid delivery unit 103 of the nucleic acid synthesizing device 10 based on the cosine similarity calculated by the similarity calculating unit 302.

<<Raman spectrum>>

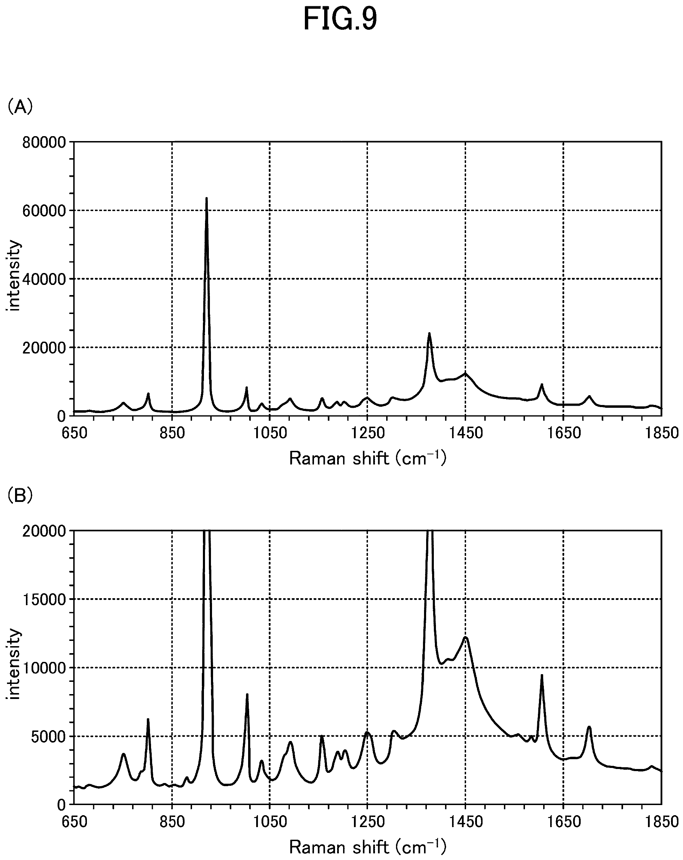

Here, a Raman spectrum will be described with reference to FIG. 9. FIG. 9 includes drawings illustrating examples of Raman spectra.

Specifically, FIG. 9 illustrates examples of Raman spectra detected from a dC nucleoside phosphoramidite. The examples in FIG. 9 are Raman spectra detected from an acetonitrile solution prepared at a concentration of 100 mM. In FIG. 9, the horizontal axis represents Raman shift, and the vertical axis represents light intensity. FIG. 9 (A) is a graph plotted in a light intensity range of from 0 through 80000, and FIG. 9 (B) is a graph plotted in a magnified light intensity range of from 0 through 20000.

<Effects of second embodiment>

The nucleoside phosphoramidite identifying system according to the present embodiment identifies the type of a nucleoside phosphoramidite based on the cosine similarity between a Raman spectrum detected from a nucleoside phosphoramidite solution and Raman spectra that have been previously detected from various types of nucleoside phosphoramidite solutions and stored as references.

Accordingly, like the first embodiment, the nucleoside phosphoramidite identifying system according to the present embodiment has a nucleoside phosphoramidite identification accuracy improved over the existing techniques.

[Third embodiment]

In the first embodiment, the control unit 306 is configured to control the nucleic acid synthesizing device 10 to stop liquid delivery when a result of checking sequence information and an identification result is a mismatch. In the third embodiment, the control unit 306 is configured to control the nucleic acid synthesizing device 10 to discard the nucleoside phosphoramidite solution being delivered.

The nucleoside phosphoramidite identifying system 1 according to the present embodiment will be described below, mainly regarding differences from the nucleoside phosphoramidite identifying system 1 according to the first embodiment.

<<Hardware configuration of nucleic acid synthesizing device>>

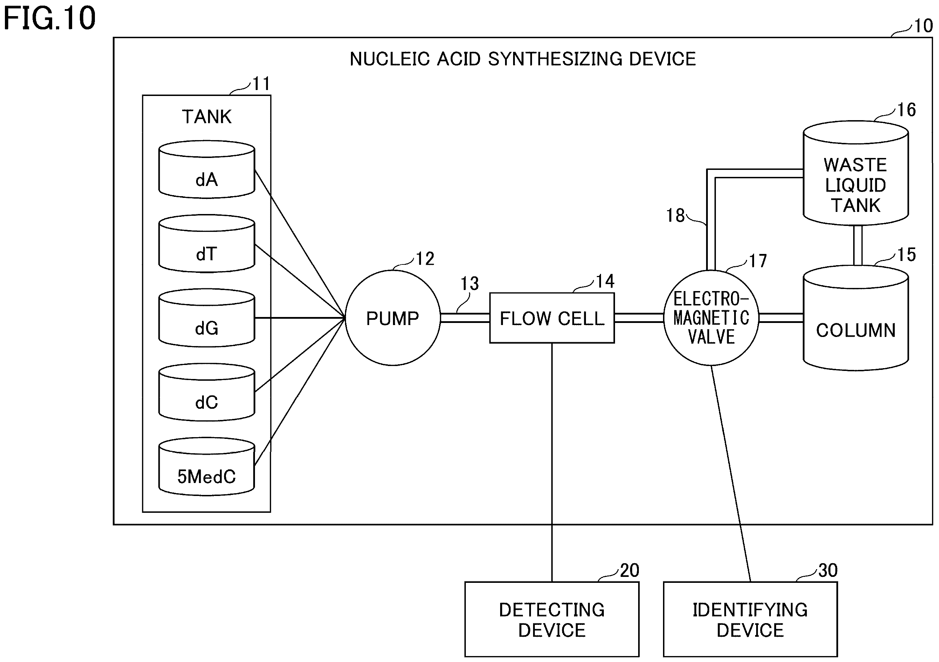

FIG. 10 is a block diagram illustrating an example of the hardware configuration of the nucleic acid synthesizing device 10 according to the present embodiment. As illustrated in FIG. 10, the nucleic acid synthesizing device 10 according to the present embodiment is different from the nucleic acid synthesizing device 10 according to the first embodiment in further including an electromagnetic valve 17 and a waste liquid tube 18.

The electromagnetic valve 17 is provided on the tube 13 that connects the flow cell 14 and the column 15. The electromagnetic valve 17 is further connected to the waste liquid tank 16 through the waste liquid tube 18. The electromagnetic valve 17 is electrically connected to the identifying device 30. The electromagnetic valve 17 is controlled to deliver a nucleoside phosphoramidite solution, which is delivered in the tube 13, to the waste liquid tube 18 in accordance with a control signal received from the identifying device 30. Hereinafter, the path that leads to the waste liquid tank 16 from the electromagnetic valve 17 through the waste liquid tube 18 may also be referred to as a "discarding path".

<Process flow of nucleoside phosphoramidite identifying method>

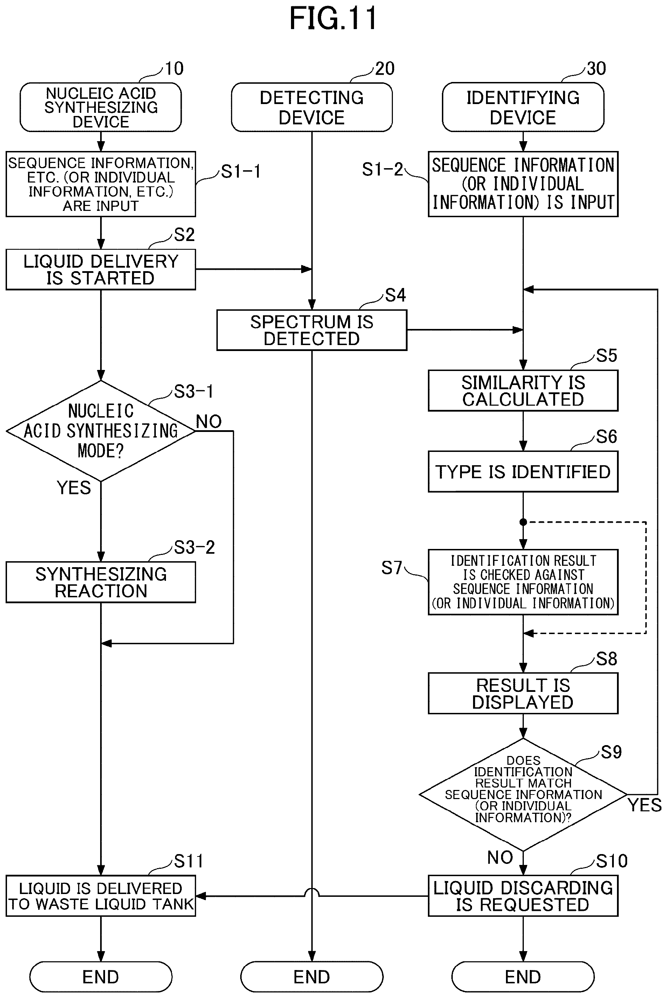

Next, the process flow of a nucleoside phosphoramidite identifying method performed by the nucleoside phosphoramidite identifying system according to the present embodiment will be described with reference to FIG. 11. FIG. 11 is a flowchart illustrating an example of the process flow of the nucleoside phosphoramidite identifying method according to the present embodiment.

As illustrated in FIG. 11, the nucleoside phosphoramidite identifying method according to the present embodiment is different from the nucleoside phosphoramidite identifying method according to the first embodiment in the processes of the steps S10 and S11.

In the step S10, the control unit 306 of the identifying device 30 controls the nucleic acid synthesizing device 10 to discard a nucleoside phosphoramidite solution. Specifically, this control is a control to send a control signal to the electromagnetic valve 17 of the nucleic acid synthesizing device 10 to request the electromagnetic valve 17 to deliver a nucleoside phosphoramidite solution to the waste liquid tube 18.

In the step S11, the liquid delivery unit 103 of the nucleic acid synthesizing device 10 delivers the nucleoside phosphoramidite solution to the waste liquid tank 16 in accordance with the control from the control unit 306. Specifically, the nucleic acid synthesizing device 10 controls the electromagnetic valve 17 such that the nucleoside phosphoramidite solution is delivered to the waste liquid tube 18.

[Example 1]

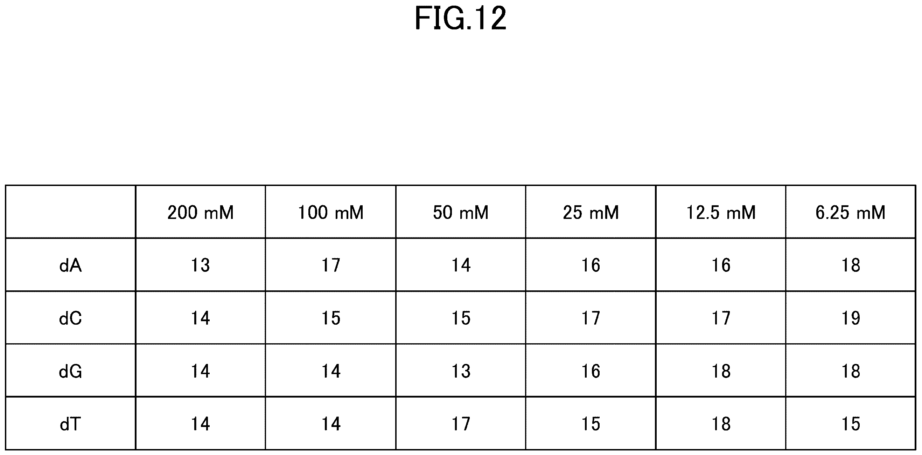

In Example 1, nucleoside phosphoramidite identification in the individual liquid delivery mode was performed using the nucleoside phosphoramidite identifying system according to the first embodiment. Example 1 will be described below with reference to FIG. 12 to FIG. 14.

A determination test was performed in the present Example and Comparative Example described below, using the same nucleic acid synthesizing device. In the determination test, a determination success rate was examined for each individual concentration level (individual liquid delivery mode), not for the type of a nucleoside phosphoramidite being synthesized (nucleic acid synthesizing mode).

<<Obtainment of references>>

In the present Example, references were previously obtained according to the following procedure. In the individual liquid delivery mode, acetonitrile solutions of nucleoside phosphoramidites (dA, dG, dT, and dC) were prepared at 25 mM, 50 mM, 100 mM, and 200 mM. Subsequently, using the nucleic acid synthesizing device, the various types of nucleoside phosphoramidite solutions were delivered to the flow cell of an infrared spectroscope (REACT IR15 (registered trademark) obtained from Mettler Toledo (registered trademark) International Inc.) connected through a plastic tube, to measure the infrared absorption spectra of the flowing acetonitrile solutions.

The measurement conditions of the infrared microscope were as follows. The measurement wavenumber range was from 1,850 $cm^{-1}$ through 650 $cm^{-1}$. The wavenumber resolution was 8 $cm^{-1}$. The number of times to add up was 16 times.

The infrared absorption spectra obtained according to the procedure described above were stored in the spectrum memory unit 300 of the identifying device 30 as references in association with individual information of the nucleoside phosphoramidites of the respective types.

<<Example 1>>

Acetonitrile solutions of nucleoside phosphoramidites (dA, dG, dT, and dC) were prepared at 6.25 mM, 12.5 mM, 25 mM, 50 mM, 100 mM, and 200 mM. Subsequently, using the nucleic acid synthesizing device, the various types of nucleoside phosphoramidite solutions were delivered to the flow cell of the infrared spectroscope mentioned above connected through a plastic tube, to measure the infrared absorption spectra of the flowing acetonitrile solutions. The measurement conditions of the infrared microscope were the same as when the references were obtained.

Data of the detected infrared absorption spectra were stored in the result memory unit 320 of the identifying device 30 in order. FIG. 12 is a diagram indicating the number of infrared absorption spectrum data detected under the conditions specified above, per solution concentration and per nucleoside phosphoramidite type.

The present Example is an example in which nucleoside phosphoramidite identification was performed based on the cosine similarity. After the infrared absorption spectra detected by the infrared spectroscope mentioned above were stored in the result memory unit 320 of the identifying device 30, each infrared absorption spectrum was converted to an index, and the cosine similarities between the index and the reference infrared absorption spectra were obtained. The type of the nucleoside phosphoramidite with which the cosine similarity was the maximum was output as the identification result.

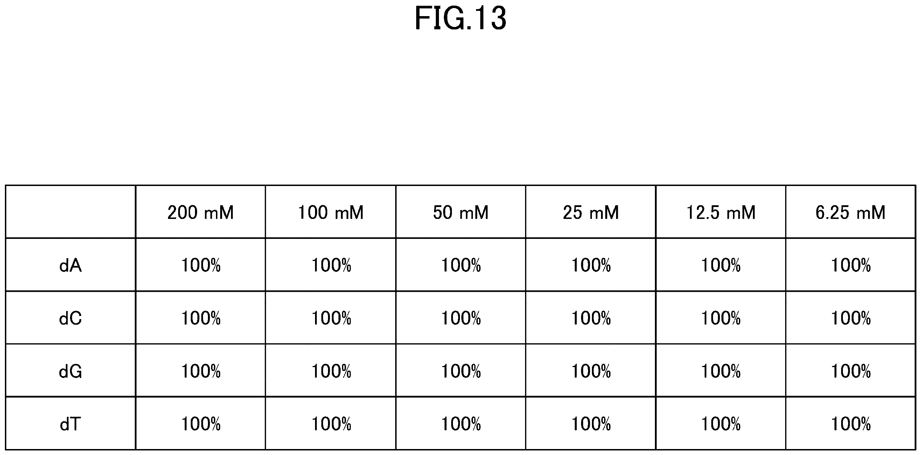

FIG. 13 is a diagram indicating the identification results in the present Example. FIG. 13 is a table indicating the determination success rate in each determination test.

<<Comparative Example 1>>

As in the present Example, previously measured infrared absorption spectra of nucleoside phosphoramidites (dA, dG, dT, and dC) were prepared as references. After infrared absorption spectra detected by the infrared spectroscope mentioned above were stored in the result memory unit 320 of the identifying device 30, the types of the nucleoside phosphoramidites of the infrared absorption spectra were determined by the PLS discrimination method.

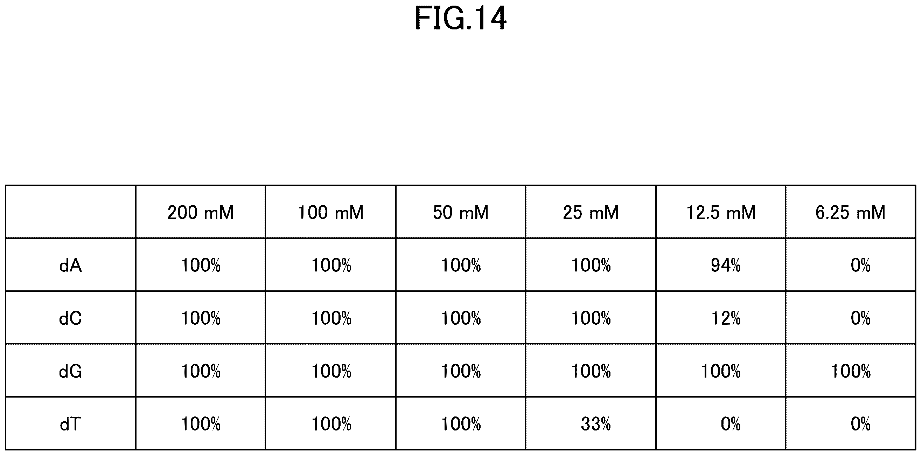

FIG. 14 is a diagram indicating the identification results in the present Comparative Example. FIG. 14 is a table indicating the determination success rate in each determination test.

<<Comparison between Example 1 and Comparative Example 1>>

As indicated in FIG. 13 and FIG. 14, all types of nucleoside phosphoramidites could be correctly identified at relatively high concentrations higher than or equal to 50 mM in both of the present Comparative Example and the present Example. On the other hand, in the present Comparative Example, erroneous identification occurred at 25 mM, and there was a tendency that the lower the concentration, the more erroneous identification cases occurred. On the other hand, in the present Example, all types of nucleoside phosphoramidites could be correctly identified even at low concentrations lower than or equal to 25 mM.

As described above, the present Example achieved a result that the identification accuracy of the nucleoside phosphoramidite identifying system according to the first embodiment was better than that of the existing method.

[Example 2]

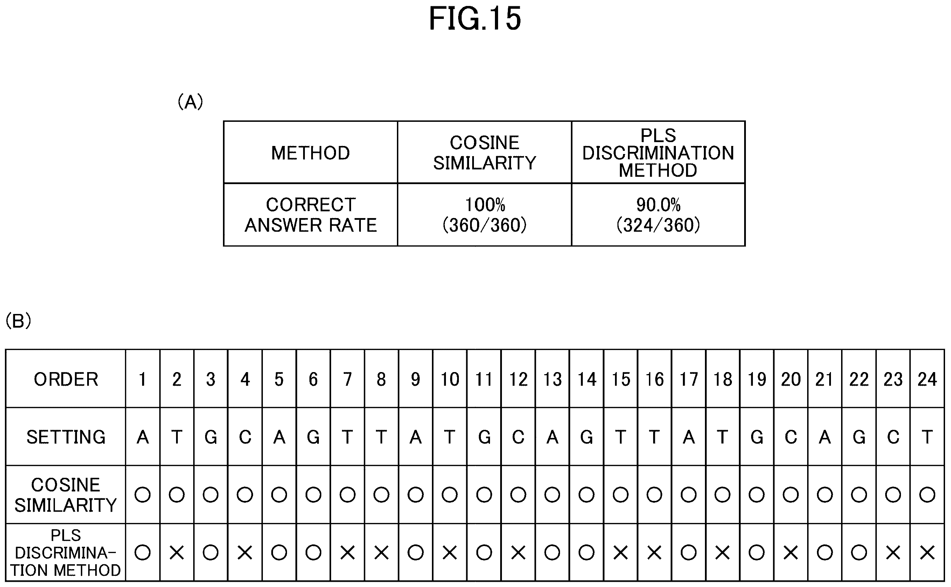

In Example 2, the type of a nucleoside phosphoramidite delivered in the nucleic acid synthesizing device was identified in the nucleic acid synthesizing mode, using the nucleoside phosphoramidite identifying system according to the first embodiment. Example 2 will be described below with reference to FIG. 15.

<<Obtainment of references>>

In the present Example, references were previously obtained according to the following procedure. In the individual liquid delivery mode, acetonitrile solutions of nucleoside phosphoramidites (dA, dG, dT, and dC) were prepared at 25 mM, 50 mM, 100 mM, and 200 mM. Subsequently, using the nucleic acid synthesizing device, the various types of nucleoside phosphoramidite solutions were delivered to the flow cell of the infrared spectroscope mentioned above connected through a plastic tube, to measure the infrared absorption spectra of the flowing acetonitrile solutions. The measurement conditions of the infrared spectroscope were the same as in Example 1. The infrared absorption spectra obtained according to the procedure described above were stored in the spectrum memory unit 300 of the identifying device 30 as references in association with individual information of the nucleoside phosphoramidites of the respective types.

<<Synthesizing method>>

In the present Example, an oligonucleotide was synthesized in the manner described below. First, porous resin beads (NITTO PHASE (registered trademark) HL UNYLINKER 350) were added by a synthesizing scale of 480 $\mu$mol into a synthesizing column (having a volume of 12.6 ml), and set on a nucleic acid synthesizing device (AKTA OLIGOPILOT 100 (registered trademark), obtained from Cytiva (registered trademark)). Next, a nucleoside phosphoramidite (200 mM) and 4,5-dicyanoimidazole serving as an activating agent were added, to allow them to undergo a coupling reaction.

The activating agent was entirely dissolved in acetonitrile, and prepared at 0.7 M. Other synthesizing reagents were as follows. DCA having a concentration of 3% in toluene was used as a deprotection agent. A 0.05 M oxidizing solution prepared exclusively for the aforementioned nucleic acid synthesizing device to be used was used as an oxidizing agent. A mixture solution of lutidine, N-methyl imidazole, and acetic anhydride in acetonitrile was used as a capping agent. The interiors of the tube and the synthesizing column were washed with acetonitrile between the coupling step, the oxidizing step, the capping step, and the deprotection step. TBA in acetonitrile (at a ratio of 8:2) was used as an amine wash reaction solution. In the nucleic acid synthesizing device, a 24merDNA oligonucleotide (5'-TCGACGTATTGACGTATTGACGTA-3', entirely oxidized: Sequence ID No. 1) was synthesized under a DMT-off condition. After the synthesis, the synthesizing column was taken out from the nucleic acid synthesizing device 10, and the oligonucleotide was cut out from the porous resin beads in the synthesizing column using an aqua ammonia, and purified, to obtain a purified product.

<<Determination test>>

While the synthesizing process described above was performed, 15 infrared absorption spectrum samples were obtained for each, through 24 synthesizing cycles. The total 360 samples were used as the targets of the determination test. In the determination test, nucleoside phosphoramidite identification was performed based on the cosine similarity with respect to each infrared absorption spectrum.

FIG. 15 (A) is a diagram indicating the identification results in the present Example. As indicated in FIG. 15, in the present Example, 360 samples out of the 360 samples were correctly identified, and all types of nucleoside phosphoramidites could be correctly identified. On the other hand, according to the existing PLS discrimination method, 324 samples out of the 360 samples were correctly identified, and 36 samples were erroneously identified.

FIG. 15 (B) was a diagram indicating the order of delivering the nucleoside phosphoramidites, and the identification results with respect to the set sequence information. A cycle in which all of the 15 samples were correctly identified is denoted as "o", and a cycle in which one or more samples were erroneously identified is denoted as "×". As indicated in FIG. 15 (B), according to the existing PLS discrimination method, erroneous identifications occurred during delivery of dT and dC.

As described above, the present Example achieved a result that the identification accuracy of the nucleoside phosphoramidite identifying system according to the first embodiment was better than that of the existing method, even for nucleoside phosphoramidites that were being synthesized.

[Example 3]

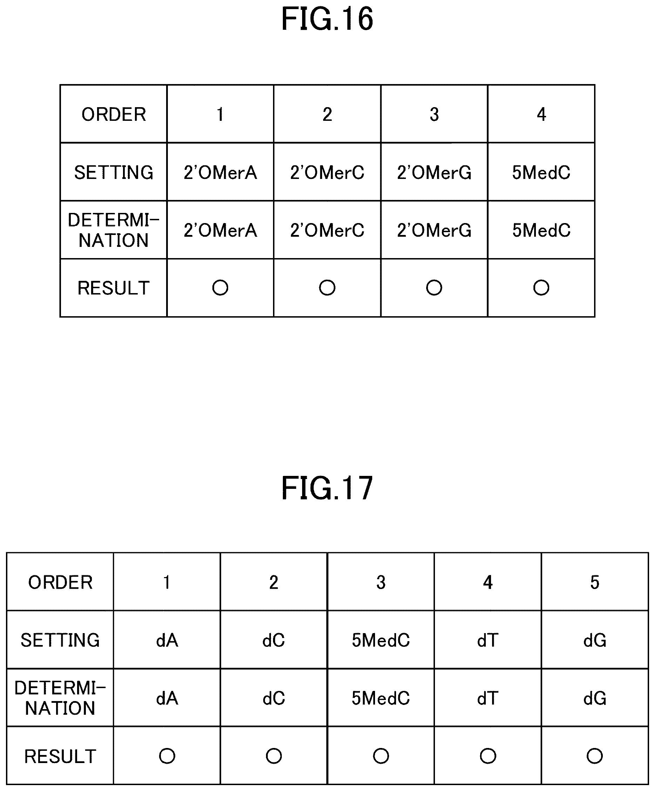

In Example 3, identification of modified nucleoside phosphoramidites in the individual liquid delivery mode was performed using the nucleoside phosphoramidite identifying system according to the first embodiment. Example 3 will be described below with reference to FIG. 16.

<<Obtainment of references>>

In the present Example, references were previously obtained according to the following procedure. In the individual liquid delivery mode, acetonitrile solutions of modified nucleoside phosphoramidites (2'OMerA, 2'OMerC, 2'OMerG, and 5MedC) were prepared at 200 mM. Subsequently, using the nucleic acid synthesizing device, the various types of modified nucleoside phosphoramidite solutions were delivered to the flow cell of the infrared spectroscope connected through a plastic tube, to measure the infrared absorption spectra of the flowing acetonitrile solutions. The measurement conditions of the infrared spectroscope were the same as in Example 1. The infrared absorption spectra obtained according to the procedure described above were stored as references in the spectrum memory unit 300 of the identifying device 30 in association with individual information of the nucleoside phosphoramidites of the respective types.

<<Determination test>>

Using the nucleic acid synthesizing device, acetonitrile solutions of four types of modified nucleoside phosphoramidites (2'OMerA, 2'OMerC, 2'OMerG, and 5MedC) prepared at 200 mM were delivered to the flow cell of the infrared spectroscope connected through a plastic tube, to measure the infrared absorption spectra of the flowing acetonitrile solutions.

After the infrared absorption spectra detected by the infrared spectroscope were stored in the result memory unit 320 of the identifying device 30, each infrared absorption spectrum was converted to an index, and the cosine similarities between the index and the reference infrared absorption spectra were obtained. The type of the modified nucleoside phosphoramidite with which the cosine similarity was the maximum was output as the identification result.

FIG. 16 is a diagram indicating the identification results in the present Example. As indicated in FIG. 16, according to the cosine similarity, all types of nucleoside phosphoramidites were correctly identified. Hence, it was indicated that not only unmodified nucleoside phosphoramidites, but also modified nucleoside phosphoramidites could be identified based on the cosine similarity. From the identification results in FIG. 16 and the identification results in Example 2 indicated in FIG. 15 (in the nucleic acid synthesizing mode), it is inferred that modified nucleoside phosphoramidites would also be correctly identified under the nucleic acid synthesizing condition (nucleic acid synthesizing mode).

[Example 4]

In Example 4, nucleoside phosphoramidite identification in the individual liquid delivery mode was performed using the nucleoside phosphoramidite identifying system according to the second embodiment (i.e., using Raman spectra). Example 4 will be described below with reference to FIG. 17.

<<Obtainment of references>>

In the present Example, references were previously obtained according to the following procedure. In the individual liquid delivery mode, acetonitrile solutions of nucleoside phosphoramidites (dA, dG, dT, dC, and 5MedC) were prepared at 11.8 mM, 23.5 mM, 36.6 mM, 48 mM, 64 mM, 80 mM, and 100 mM. Subsequently, the various types of nucleoside phosphoramidite solutions were delivered to a flow cell-integrated probe (obtained from Marq Metrix) of a Raman spectroscope (PI-200 obtained from Process Instruments Inc.) connected through a plastic tube using a single plunger pump (SP-21 obtained from Flom, Inc.), to measure Raman spectra of the flowing acetonitrile solutions.

The measurement conditions of the Raman spectroscope were as follows. The laser wavelength was 532 nm. The measurement wavenumber range was from 1,850 $cm^{-1}$ through 650 $cm^{-1}$. The wavenumber resolution was 8 $cm^{-1}$. The light exposure time was 1 second. The number of times to add up was 10 times.

The Raman spectra obtained according to the procedure described above were stored in the spectrum memory unit 300 of the identifying device 30 as references in association with individual information of the nucleoside phosphoramidites of the respective types.

<<Determination test>>

Acetonitrile solutions of nucleoside phosphoramidite (dA, dC, 5MedC, dT, and dG) were prepared at 200 mM, and then set in a duct of the nucleic acid synthesizing device. The five types of nucleoside phosphoramidites were delivered to the flow cell-integrated probe of the Raman spectroscope in the order of dA, dC, 5MedC, dT, and dG under software setting of the nucleic acid synthesizing device.

When switching between nucleoside phosphoramidites, the interior of the plastic tube was washed and purged with acetonitrile (12 mL). The cosine similarities between the Raman spectra obtained from the flow cell and the Raman spectra registered as references were obtained. The type of the nucleoside phosphoramidite with which the cosine similarity was the maximum was output as the identification result.

FIG. 17 is a diagram indicating the identification results in the present Example. As indicated in FIG. 17, the five types of nucleoside phosphoramidites were delivered, and the types of all of the nucleoside phosphoramidites were correctly identified. From the Raman spectrum identification results in FIG. 17 and the identification results in Example 2 indicated in FIG. 15 (likewise using the cosine similarity in the nucleic acid synthesizing mode), it is inferred that all types of nucleoside phosphoramidites would be correctly identified also under the nucleic acid synthesizing condition (nucleic acid synthesizing mode) based on Raman spectrum detection and cosine similarity.

[Supplemental]

Each function of the embodiments described above can be realized by one or a plurality of processing circuits. In the present specification, a "processing circuit" encompasses devices such as a processor programmed by software to execute each function, such as a processor implemented on an electronic circuit, and an Application Specific Integrated Circuit (ASIC), a Digital Signal Processor (DSP), a Field Programmable Gate Array (FPGA), and existing circuit modules that are designed to execute each function described above.

The embodiments of the present invention have been described above in detail. However, the present invention is not limited to these embodiments, and various modifications or changes can be applied to the present invention within the scope of the spirit of the present invention described in the claims.

The present application claims priority to Japanese Patent Application No. 2022-68370 filed with Japan Patent Office on April 18, 2022, the entire content of which is incorporated herein by reference.

Reference Signs List

1:       nucleoside phosphoramidite identifying system
10:      nucleic acid synthesizing device
101:     sequence information input unit
102:     liquid storage unit
103:     liquid delivery unit
104:     synthesizing unit
20:      detecting device
201:     detecting unit
30:      identifying device
300:     spectrum memory unit
301:     sequence information input unit
302:     similarity calculating unit
303:     identifying unit
304:     checking unit
305:     result display unit
306:     control unit
310:     sequence information memory unit
320:     result memory unit

**Claims**

1. A nucleoside phosphoramidite identifying system, comprising:

   a memory unit configured to store spectra of solutions of a plurality of different nucleoside phosphoramidites;
   a detecting unit configured to detect a spectrum of a solution of a nucleoside phosphoramidite; and
   an identifying unit configured to identify the nucleoside phosphoramidite based on cosine similarity between the spectra stored in the memory unit and the spectrum detected by the detecting unit.

2. The nucleoside phosphoramidite identifying system according to claim 1, further comprising:

   a liquid delivery unit configured to deliver the solutions of the nucleoside phosphoramidites; and
   a synthesizing unit configured to react the nucleoside phosphoramidites, to synthesize an oligonucleotide,
   wherein the detecting unit detects the spectrum from the solution of the nucleoside phosphoramidite delivered by the liquid delivery unit.

3. The nucleoside phosphoramidite identifying system according to claim 2,
   wherein the detecting unit detects the spectrum of the solution of the nucleoside phosphoramidite delivered between the liquid delivery unit and the synthesizing unit.

4. The nucleoside phosphoramidite identifying system according to claim 3,
   wherein the detecting unit detects the spectrum of the solution of the nucleoside phosphoramidite by infrared spectroscopy or Raman spectroscopy.

5. The nucleoside phosphoramidite identifying system according to claim 2, further comprising:

   an input unit configured to receive an input of sequence information of the oligonucleotide; and
   a checking unit configured to check the sequence information and an identification result of the identifying unit against each other.

6. The nucleoside phosphoramidite identifying system according to claim 5, further comprising:
a display unit configured to display the identification result of the identifying unit and a checking result of the checking unit in association with each other.

7. The nucleoside phosphoramidite identifying system according to claim 6, further comprising:
a control unit configured to perform a control to stop the liquid delivery unit when the checking result includes an error.

8. The nucleoside phosphoramidite identifying system according to claim 6, further comprising:
a control unit configured to perform a control to deliver the solution of the nucleoside phosphoramidite to a discarding path when the checking result includes an error.

9. The nucleoside phosphoramidite identifying system according to claim 2,
wherein the solution of the nucleoside phosphoramidite has a concentration of 25 mM or lower.

10. A nucleoside phosphoramidite identifying method by which a computer executes:

a storing procedure for storing spectra of solutions of a plurality of different nucleoside phosphoramidites;
a detecting procedure for detecting a spectrum of a solution of a nucleoside phosphoramidite; and
an identifying procedure for identifying the nucleoside phosphoramidite based on cosine similarity between the spectra stored in the storing procedure and the spectrum detected in the detecting procedure.

11. A program causing a computer to execute:

a storing procedure for storing spectra of solutions of a plurality of different nucleoside phosphoramidites;
a detecting procedure for detecting a spectrum of a solution of a nucleoside phosphoramidite; and
an identifying procedure for identifying the nucleoside phosphoramidite based on cosine similarity between the spectra stored in the storing procedure and the spectrum detected in the detecting procedure.

# FIG.1

1 NUCLEOSIDE PHOSPHORAMIDITE IDENTIFYING SYSTEM

10

NUCLEIC ACID
SYNTHESIZING SYSTEM

20

DETECTING DEVICE

30

IDENTIFYING DEVICE

FIG.2

EP 4 513 170 A1

# FIG.3

EP 4 513 170 A1

# FIG.4

# FIG.5

| NUCLEIC ACID SYNTHESIZING DEVICE (10) | DETECTING DEVICE (20) | IDENTIFYING DEVICE (30) |
|---|---|---|
| SEQUENCE INFORMATION, ETC. (OR INDIVIDUAL INFORMATION, ETC.) ARE INPUT (S1-1) | | SEQUENCE INFORMATION (OR INDIVIDUAL INFORMATION) IS INPUT (S1-2) |
| LIQUID DELIVERY IS STARTED (S2) | SPECTRUM IS DETECTED (S4) | SIMILARITY IS CALCULATED (S5) |
| NUCLEIC ACID SYNTHESIZING MODE? (S3-1) | | TYPE IS IDENTIFIED (S6) |
| SYNTHESIZING REACTION (S3-2) | | IDENTIFICATION RESULT IS CHECKED AGAINST SEQUENCE INFORMATION (OR INDIVIDUAL INFORMATION) (S7) |
| | | RESULT IS DISPLAYED (S8) |
| | | DOES IDENTIFICATION RESULT MATCH SEQUENCE INFORMATION (OR INDIVIDUAL INFORMATION)? (S9) |
| LIQUID DELIVERY IS STOPPED (S11) | | LIQUID DELIVERY STOP IS REQUESTED (S10) |
| END | END | END |

FIG.6

# FIG.7

ELAPSED TIME    5:01

| SEQUENCE INFORMATION | A | 5MedC | C | T | G |

| IDENTIFICATION RESULT | A | 5MedC | T | – | – |

Start

Cancel

1004-1    1004-3    1004-2

# FIG.8

EP 4 513 170 A1

# FIG.9

(A)

(B)

# FIG.10

NUCLEIC ACID SYNTHESIZING DEVICE 10

TANK 11

dA

dT

dG

dC

5MedC

PUMP 12

13

FLOW CELL 14

ELECTRO-MAGNETIC VALVE 17

18

WASTE LIQUID TANK 16

COLUMN 15

DETECTING DEVICE 20

IDENTIFYING DEVICE 30

EP 4 513 170 A1

# FIG.11

## FIG.12

|  | 200 mM | 100 mM | 50 mM | 25 mM | 12.5 mM | 6.25 mM |
|---|---|---|---|---|---|---|
| dA | 13 | 17 | 14 | 16 | 16 | 18 |
| dC | 14 | 15 | 15 | 17 | 17 | 19 |
| dG | 14 | 14 | 13 | 16 | 18 | 18 |
| dT | 14 | 14 | 17 | 15 | 18 | 15 |

# FIG.13

|  | 200 mM | 100 mM | 50 mM | 25 mM | 12.5 mM | 6.25 mM |
|---|---|---|---|---|---|---|
| dA | 100% | 100% | 100% | 100% | 100% | 100% |
| dC | 100% | 100% | 100% | 100% | 100% | 100% |
| dG | 100% | 100% | 100% | 100% | 100% | 100% |
| dT | 100% | 100% | 100% | 100% | 100% | 100% |

# FIG.14

|     | 200 mM | 100 mM | 50 mM | 25 mM | 12.5 mM | 6.25 mM |
|-----|--------|--------|-------|-------|---------|---------|
| dA  | 100%   | 100%   | 100%  | 100%  | 94%     | 0%      |
| dC  | 100%   | 100%   | 100%  | 100%  | 12%     | 0%      |
| dG  | 100%   | 100%   | 100%  | 100%  | 100%    | 100%    |
| dT  | 100%   | 100%   | 100%  | 33%   | 0%      | 0%      |

# FIG.15

(A)

| METHOD | COSINE SIMILARITY | PLS DISCRIMINATION METHOD |
|---|---|---|
| CORRECT ANSWER RATE | 100% (360/360) | 90.0% (324/360) |

(B)

| ORDER | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 |
|---|---|---|---|---|---|---|---|---|---|---|---|---|---|---|---|---|---|---|---|---|---|---|---|---|
| SETTING | A | T | G | C | A | G | T | T | A | T | G | C | A | G | T | T | A | T | G | C | A | G | C | T |
| COSINE SIMILARITY | O | O | O | O | O | O | O | O | O | O | O | O | O | O | O | O | O | O | O | O | O | O | O | O |
| PLS DISCRIMINA-TION METHOD | O | X | O | X | O | O | X | X | O | X | O | X | O | O | X | X | O | X | O | X | O | O | X | X |

EP 4 513 170 A1

# FIG.16

| ORDER | 1 | 2 | 3 | 4 |
|---|---|---|---|---|
| SETTING | 2'OMerA | 2'OMerC | 2'OMerG | 5MedC |
| DETERMI-NATION | 2'OMerA | 2'OMerC | 2'OMerG | 5MedC |
| RESULT | ○ | ○ | ○ | ○ |

# FIG.17

| ORDER | 1 | 2 | 3 | 4 | 5 |
|---|---|---|---|---|---|
| SETTING | dA | dC | 5MedC | dT | dG |
| DETERMI-NATION | dA | dC | 5MedC | dT | dG |
| RESULT | ○ | ○ | ○ | ○ | ○ |

# INTERNATIONAL SEARCH REPORT

| International application No. |
| --- |
| **PCT/JP2023/014606** |

## A. CLASSIFICATION OF SUBJECT MATTER

*G01N 21/3577*(2014.01)i; *G01N 21/65*(2006.01)i
FI:   G01N21/3577; G01N21/65

According to International Patent Classification (IPC) or to both national classification and IPC

## B. FIELDS SEARCHED

Minimum documentation searched (classification system followed by classification symbols)

G01N21/3577; G01N21/65

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII)

## C. DOCUMENTS CONSIDERED TO BE RELEVANT

| Category* | Citation of document, with indication, where appropriate, of the relevant passages | Relevant to claim No. |
| --- | --- | --- |
| Y | US 2018/0059011 A1 (OPTIONLINE LLC) 01 March 2018 (2018-03-01)<br>claims 1-7, paragraph [0002] | 1-11 |
| Y | MCELDERRY et al. In-line Phosphoramidite Identification by FTIR to Support Real-Time Oligonucleotide Sequence Confirmation. Organic Process Research & Development. 2021, vol. 25, issue 2, pp. 262-270<br>abstract, p. 262, right column, line 10 to p. 264, right column, line 8, fig. 1 | 1-11 |
| A | HE et al. Simultaneous Quantification of Nucleosides and Nucleotides from Biological Samples. Journal of the American Society for Mass Spectrometry. 2019, vol. 30, issue 6, pp. 987-1000<br>abstract, p. 989, data analysis | 1-11 |
| A | GAUTAM et al. Review of multidimensional data processing approaches for Raman and infrared spectroscopy. EPJ Techniques and Instrumentation. 2015, vol. 2, no. 8, pp. 1-38<br>abstract | 1-11 |
| A | JP 2021-177130 A (GUNMA PREF.) 11 November 2021 (2021-11-11) | 1-11 |

☐ Further documents are listed in the continuation of Box C.        ☑ See patent family annex.

| * Special categories of cited documents: | |
| --- | --- |
| "A" document defining the general state of the art which is not considered to be of particular relevance | "T" later document published after the international filing date or priority date and not in conflict with the application but cited to understand the principle or theory underlying the invention |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **12 June 2023** | **20 June 2023** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2023/014606**

| Patent document cited in search report | | | Publication date (day/month/year) | Patent family member(s) | | | Publication date (day/month/year) |
|---|---|---|---|---|---|---|---|
| US | 2018/0059011 | A1 | 01 March 2018 | WO | 2018/035592 | A1 | |
| | | | | CA | 3034957 | A1 | |
| JP | 2021-177130 | A | 11 November 2021 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2020210476 A

- JP 2022068370 A

**Non-patent literature cited in the description**

- **JOHN-DAVID MCELDERRY** ; **DANIEL HILL** ; **ELLIOTT SCHMITT** ; **XIAOYE SU** ; **JESSICA STOLEE**. In-line Phosphoramidite Identification by FTIR to Support Real-Time Oligonucleotide Sequence Confirmation. *Organic Process Research & Development*, 2021, vol. 25 (2), 262-270